# EUROPEAN PATENT SPECIFICATION

(11) **EP 4 391 964 B1**
(45) Date of publication and mention of the grant of the patent: **08.04.2026**
(21) Application number: 21834977.7
(22) Date of filing: 30.11.2021
(51) Int. Cl.: A61F 2/00, A61F 2/48

(54) **A URETHRAL CUFF FOR USE IN ARTIFICIAL URINARY SPHINCTERS**
URETHRALE MANSCHETTE ZUR VERWENDUNG IN KÜNSTLICHEN SCHLIESSMUSKELN
MANCHON URÉTRAL DESTINÉ À ÊTRE UTILISÉ DANS DES SPHINCTERS URINAIRES ARTIFICIELS

(43) Date of publication of application: 03.07.2024
(73) Proprietor: Lüleci, Hüseyin, 34805 Beykoz/Istanbul (TR)
(72) Inventor: LÜLECI, Ahmet Melih, 34805 Beykoz/Istanbul (TR); LÜLECI, Hüseyin, 34805 Beykoz/Istanbul (TR)
(74) Representative: Genç Ilhan, Oznur
(86) International application number: PCT/TR2021/051319
(87) International publication number: WO 2023/101621

(56) References cited:
- EP-A1- 2 815 720
- WO-A1-2021/224719
- DE-A1- 2 438 691
- US-A- 3 866 611
- US-A- 4 786 276
- US-A1- 2019 328 501

## Description

### TECHNICAL FIELD OF THE INVENTION

The present invention relates to an improved urethral cuff for use in artificial urinary sphincters (AUS) for treating incontinence.

### BACKGROUND OF THE INVENTION

A biological urinary sphincter prevents urinary flow via mucosal coaptation, compression and pressure transmission. On the other hand, an artificial urinary sphincter mimics the biological urinary sphincter by providing a competent bladder outlet during urinary storage and an open unobstructed outlet to permit voluntary urination.

A known treatment for some cases of incontinence is to provide a patient with a mechanism to occlude the affected excretory body passage. These mechanisms are typically surgically implanted within the patient's body and are adapted to be operable by the patient to selectively open and occlude the body passage. Inflatable artificial sphincters are well known devices in the state of the art. Inflatable sphincters typically include an inflatable cuff for surrounding the passage to be occluded. Usually, a pump cooperatively associated with a fluid reservoir is utilized to transfer fluid into and out of the cuff. As fluid is transferred into the cuff, the cuff inflates and occludes the circumscribed body passage.

Artificial urinary sphincters (AUS) known in the state of the art consist of three major parts, namely a fluid reservoir, a cuff and a pump which is usually designated as the control mechanism of the AUS. The pump can be placed in a man's scrotum. It can also be placed underneath the skin in a woman's lower belly, labia or leg. Two conduit tubes connect all three major parts to each other. Use of an extra element, in particular of a conduit tube, increases the implantation time, complexity of the surgery and most importantly, the infection risk of a patient after implantation within the body.

Many conventional sphincteric mechanisms, namely cuffs, are characterized by a circumferential occlusion geometry. However, these cuffs configured to apply circumferential occlusive pressures fail to efficiently occlude the excretory body passage when a sudden pressure increase occurs in the patient's abdomen. A sudden pressure increase may occur when, for instance, the patient laughs, coughs or is burst into laughter and also by way of certain physical movements such as bending the upper body down or when lifting a weight. In such cases, the normal pressure formed in the inflatable cuff may fail to effectively occlude the excretory body passage and excreted fluid which already accumulated behind the cuff or in the bladder may unintentionally leak outside the patient's body. In addition, it is a known fact that circumferential cuff applications increase the susceptibility of the urethra to atrophy or erosion. US4584990A, for instance, draws attention to the inefficacies of sphincter structures that apply circumferential occlusive pressure and aims to develop a structure that eliminates these disadvantages to provide an efficient continence for the patient. Accordingly, the proposed occlusion cuff includes a pair of oppositely disposed, inflatable patient-activated and physician-controlled chambers. A fluid medium is selectively supplied to one or both of the chambers for causing expansions thereof and an increase in the occlusive pressures applied to the lumen. It mentions that the oppositely disposed chambers apply a minimal diametric occlusive pressure to the lumen to more effectively and efficiently achieve coaptation with a reduced possibility of atrophy, erosion and/or ischemia. However, the two-chamber mechanism proposed here applies a combined pressure in such a way as to shrink a radially narrow area of the lumen. Since the occlusive pressure remains in a single plane, there is still a risk that the fluid passage cannot be completely blocked. More importantly, a limited area of occlusion can also cause excessive compression of the capillaries in the lumen and thus resulting in damage to the living tissue. Moreover, this mechanism requires both the patient and a physician interference, therefore it needs to be simplified in order to avoid complexity and to enhance patient compliance. It also needs to be improved to ensure the efficacity in preventing incontinence.

US2019/0328501 A1, relates to a tissue compression device. The tissue compression device includes a pump, a liquid storage chamber, catheters, and a compression device. The liquid storage chamber is coupled to the pump, the liquid storage chamber stores fluid of a preset volume, the liquid storage chamber communicates with the compression device via the catheters.

EP2815720 (A1) discloses an artificial urinary sphincter (AUS) system including a pump attachable with tubing between a pressure regulating reservoir and a cuff. The cuff is configured for placement around a portion of a urethra and has a frame with at least one inflatable balloon attached to the frame.

US4786276 (A) discloses a sphincteric web for the treatment of incontinence having a particular alignment of three inflatable pressure cushions by which to prosthetically replicate the geometry of a normally continent rectum. The sphincteric web surrounds and embraces a patient's colon (or rectum), and the pressure cushions are inflated with hydraulic fluid from a hypodermically accessible source thereof.

US2019/0328501 A1 discloses an implantable prosthetic clamp, e.g. to treat urinary incontinence, comprises a compressible fluid reservoir, a jaw movable between open and closed positions and biased towards one, and a jaw actuator connected by a tube to the reservoir. The jaw has three spaced parallel arms connected by a bight and arranged at the spices of an equilateral triangle when viewed in cross-section.

US3866611 (A) relates a penile clamp for control of male incontinence including a pair of clamping surfaces hingedly connected together at one end by a flexible hinge member and releasably attachable together at their opposite end by a self-adhering type of fastening, with a pair of pressure applying members in spaced-apart relation on one clamping surface and a single pressure applying member on the opposite clamping surface disposed in relation to the pair of opposing pressure members to exert a three-point clamping action on an organ clamped between the surface.

WO2021224719 (A1) relates to a cuff of artificial urethral sphincter. The cuff comprises an inflatable chamber with a pair of opposite main walls and two side walls, wherein first end portions of the main walls and side walls form a first end of the inflatable chamber and second end portions of the main walls and side walls form a second end of the inflatable chamber opposite the first end.

Conventional urethral cuffs known in the state of the art pressurize the urethral canal circumferentially and continence can be established only when a high pressure is applied on the periphery of the urethra. The high peripheric pressure, however, is harmful to the urethra since the urethral canal is a living tissue and the high pressure also results in occlusion of the capillary vessels within the urethra at the plane where peripheric pressure is applied. There are reported cases where the urethra of the patient was physically harmed and was partially torn due to the high local pressure which was circumferentially applied.

### OBJECTS OF THE PRESENT INVENTION

Primary object of the present invention is to provide a new urethral cuff which eliminates the drawbacks of the existing cuffs uses in artificial urinary sphincters.

In particular, an object of the present invention is to provide a new occluding mechanism for a urethral cuff which improves the efficacity in overcoming incontinence without damaging the capillaries in the urethra and the corresponding tissue.

A further object of the present invention is to provide a urethral cuff providing enhanced patient compliance and enhanced incontinence security.

A further object of the present invention is to provide a simplified urethral cuff which is easy to manufacture.

A further object of the present invention is to provide a urethral cuff which allows insertion of an endoscope or a resectoscope while deflated.

A final object of the present invention is to provide a simplified urethral cuff which is easy to implant.

### BRIEF DESCRIPTIONS OF THE DRAWINGS

The figures whose brief explanations are herewith provided are solely intended for providing a better understanding of the present invention and are as such not intended to define the scope of protection or the context in which said scope is interpreted in the absence of the description.
Fig. 1 shows a perspective view of an artificial urinary sphincter of the prior art
Fig. 2 shows the top view of the urethral cuff according to the present invention, in the inflated and open state of the urethral cuff
Fig. 3 shows the top view of the urethral cuff given in Fig. 2, in the deflated and open state of the urethral cuff
Fig. 4 shows the front view of a urethral cuff according to the present invention, holding a urethra, in the inflated and folded state of the urethral cuff
Fig. 5 shows a front cross-sectional view of the urethral cuff given in Fig. 4, holding a urethra, in the inflated and folded state of the urethral cuff
Fig. 6 shows the side view of the urethral cuff according to the present invention, holding a urethra, holding a urethra, in the inflated and folded state of the urethral cuff
Fig. 7 shows the side view of the urethral cuff given in Fig. 6, holding a urethra, in the deflated and folded state of the urethral cuff
Fig. 8 shows a top perspective view of the urethral cuff according to the present invention, in the inflated and open state of the urethral cuff
Fig. 9 shows a top perspective view of the urethral cuff given in Fig. 8, in the deflated and open state of the urethral cuff

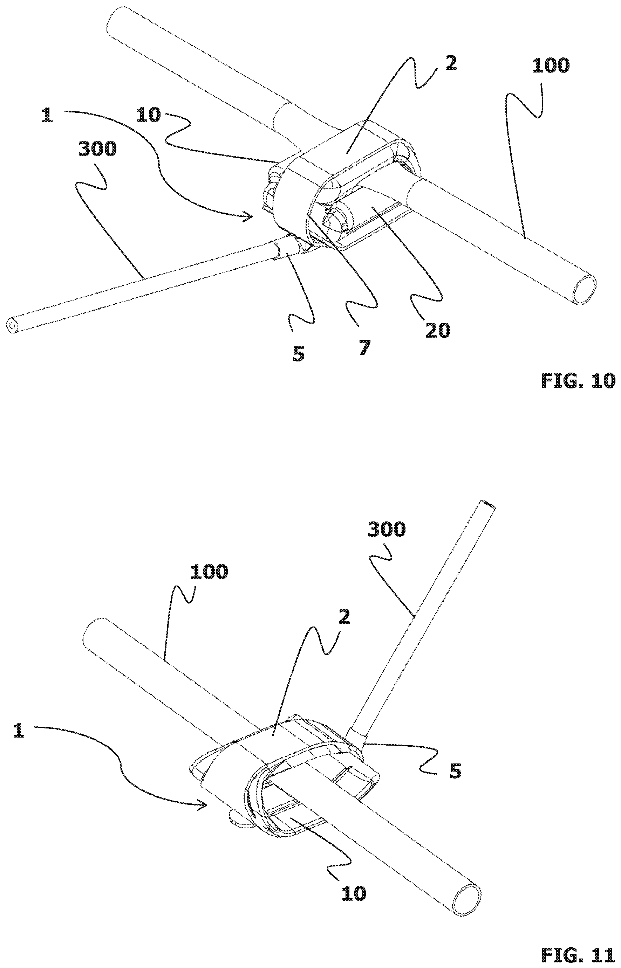

Fig. 10 shows a top perspective view of the urethral cuff according to the present invention, holding a urethra, in the inflated and folded state of the urethral cuff
Fig. 11 shows a top perspective view of the urethral cuff given in Fig. 10, holding a urethra, in the deflated and folded state of the urethral cuff
Fig. 12 shows a top view of the urethral cuff according to the present invention, holding a urethra, in the inflated and folded state of the urethral cuff
Fig. 13 shows a top view of the urethral cuff given in Fig. 12, holding a urethra, in the deflated and folded state of the urethral cuff
Fig. 14 shows a bottom perspective view of an embodiment of the urethral cuff according to the present invention in the inflated and folded state of the urethral cuff
Fig. 15 shows a bottom perspective view of another embodiment of the urethral cuff according to the present invention in the inflated and folded state of the urethral cuff

### DETAILED DESCRIPTION OF THE INVENTION

The list of reference numerals used in the appended drawings is as follows;
- 1.: Urethral cuff
- 2.: Backsheet
21. First portion
22. Second portion
- 3.: Gap
- 4.: Recess
- 5.: Inlet
51. Protrusion
52. Inlet hole
- 6.: Folding section
61. Conduit
- 7.: Pull tab
71. Connection hole
- 8.: Reinforcement element
- 10.: First inflatable cushion
- 20.: Second inflatable cushion
- 30.: Third inflatable cushion
- 40.: Fourth inflatable cushion
- 100.: Urethra
- 200.: Length
- 300.: Tube
- 400.: Pump
- 500.: Fluid reservoir
- 600.: Cuff of the prior art

Objects of the present invention are achieved by the features of Claim 1 in which a urethral cuff (1) for use in an artificial urinary sphincter is disclosed. The urethral cuff (1) has a backsheet (2) forming the body of the urethral cuff (1) and composed of a first portion (21) and a second portion (22). The first portion (21) has a first inflatable cushion (10) and a second inflatable cushion (20) which are separated from each other by a gap (3). The second portion (22) has a third inflatable cushion (30) which is located such that said third inflatable cushion (30) corresponds on top of said gap (3) when the second portion (22) is folded on top of the first portion (21) such that when the urethral cuff (1) is implanted around the urethra (100) of a patient and all three inflatable cushions (10, 20, 30) are inflated, a length (200) of the urethra (100) is flattened among the inflatable cushions (10, 20, 30) resulting in occlusion of the urethra (100).

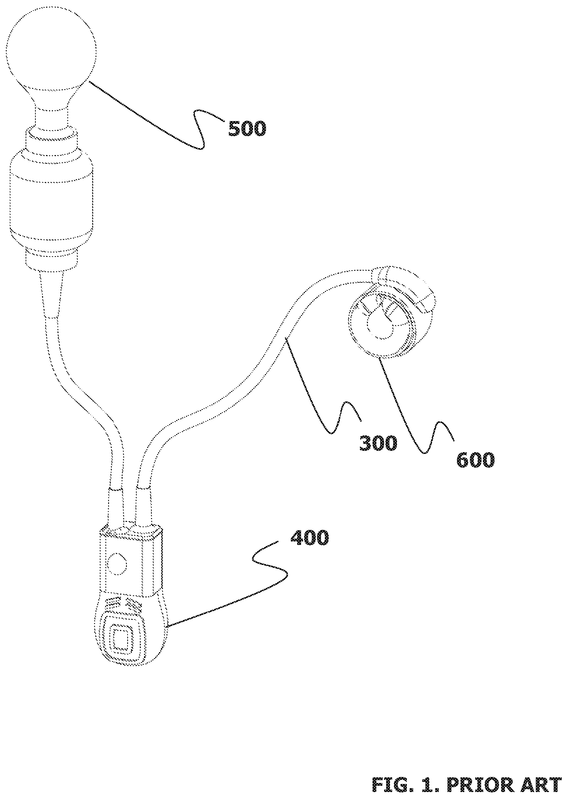

An artificial urinary sphincter of the prior art comprising a pump (400), a fluid reservoir (500) and a cuff (600) is given in Fig. 1. The cuff (600) shown here is configured to diametrically shrink and occlude the urethra unlike the present invention in which a urethral cuff (1) occluding the urethra (100) by flattening a length (200) of the urethra (100) is proposed.

The advantage of the occluding mechanism according to the present invention is that since the urethra (100) is compressed to be flattened along a certain length (200), the urethral cuff (1) wraps the urethra (100) more firmly but with a more homogeneous distribution of pressure thus preventing urinary incontinence more effectively while providing an enhanced patient compliance.

Moreover, when the urethral cuff (1) according to the present invention is implanted around the urethra (100) of a patient and all three inflatable cushions (10, 20, 30) are inflated, the urethra (100) is flattened at least in three different planes. These planes form certain angles with each other such that the flattened length (200) elongates with multiple breaking points and/or curved sections. This configuration allows the area of the flattened length (200) to be expanded, thus reducing the pressure applied per area even more while increasing the occlusion of the urethra (100). Furthermore, as the urethral cuff (100) is inflated around the urethra (100), all three inflatable cushions (10, 20, 30) inflate simultaneously, thus avoiding instantaneous local high pressure on the urethra (100).

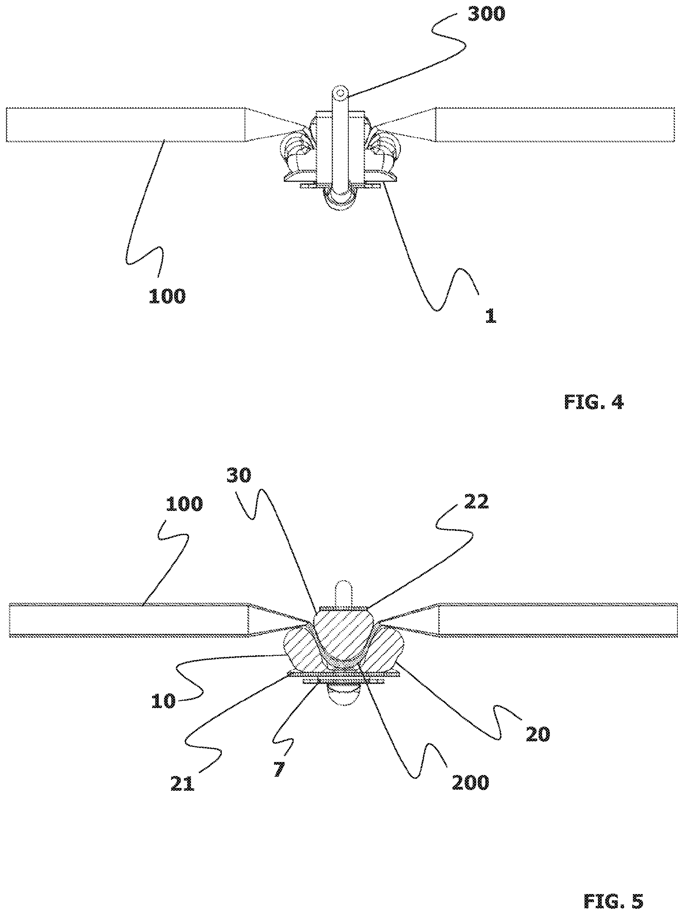

Fig. 4 shows a front view of a urethral cuff according to the present invention in a state as implanted around the urethra (100) and in its inflated state. This configuration enables the urethra (100) to narrow first from both sides approaching into the urethral cuff (1) and then to be completely flattened. Thus, the amount of the urethral fluid per length is reduced before it reaches the flattened length (200). This process also plays a role in ensuring continence. The flattened length (200) of the urethra (100) can be U-shaped as depicted in Fig. 5 or the length (200) can also be flattened so as to form a sinusoidal- wave shape.

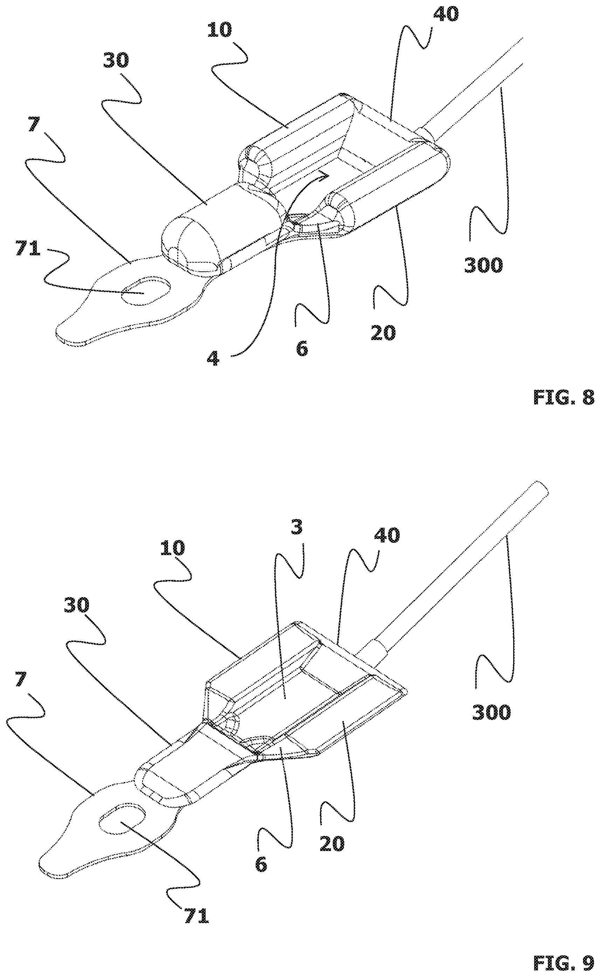

According to the present invention, the first portion (21) further comprises a fourth inflatable cushion (40). The fourth inflatable cushion (40) connects the first and second inflatable cushions (10, 20) such that the two-dimensional gap (3) converts into a three-dimensional recess (4) when all four inflatable cushions (10, 20, 30, 40) are inflated. In this case, the third inflatable cushion (30) fits into and fills said recess (4) when the second portion (22) is folded on top of the first portion (21). As can be seen in Fig. 8, the recess (4) surrounded by the first, second and fourth inflatable cushions (10, 20, 40) is in the form of a housing in which the third inflatable cushion (30) can be fully seated. The third inflatable cushion (30) is sized and shaped to fit the recess (4) in the inflated state of the three cushions (10, 20, 30).

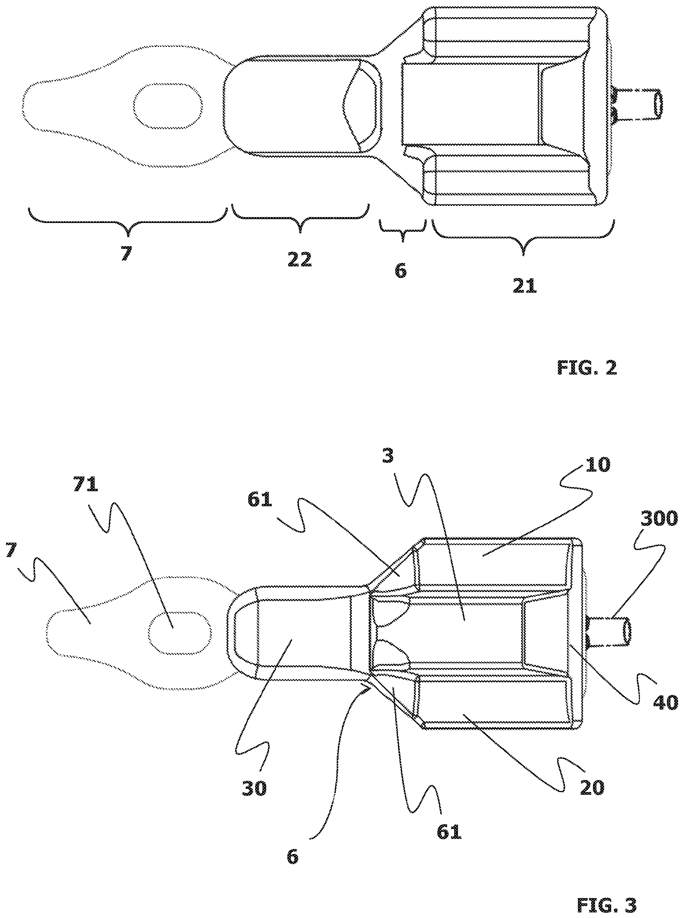

The urethral cuff (1) further comprises a folding section (6) that allows fluid communication between the first portion (21) and the second portion (22) by means of at least one conduit (61), thus allowing simultaneous inflation or deflation of all four inflatable cushions (10, 20, 30, 40). The folding section (6) is in the form of a bridge between the first portion (21) and the second portion (22), enabling the second portion (22) to be folded on top of the first portion (21) without creating an unintentional tension on the urethra (100). The folding section (6) is configured to be aligned with the third inflatable cushion (30) as it extends from the first portion (21) to the second portion (22) as depicted in Fig. 2 and Fig. 3. Said folding section (6) preferably contains two conduits (61), such that each conduit (61) is extending between the third inflatable cushion (30) and each of the first and second inflatable cushions (10, 20). Said conduits (61) ensure the simultaneous transfer of the pressurizing fluid to all three inflatable cushions (10, 20, 30), preferably to all four inflatable cushions (10, 20, 30, 40), regardless of where the pressurizing fluid enters into the urethral cuff (1).

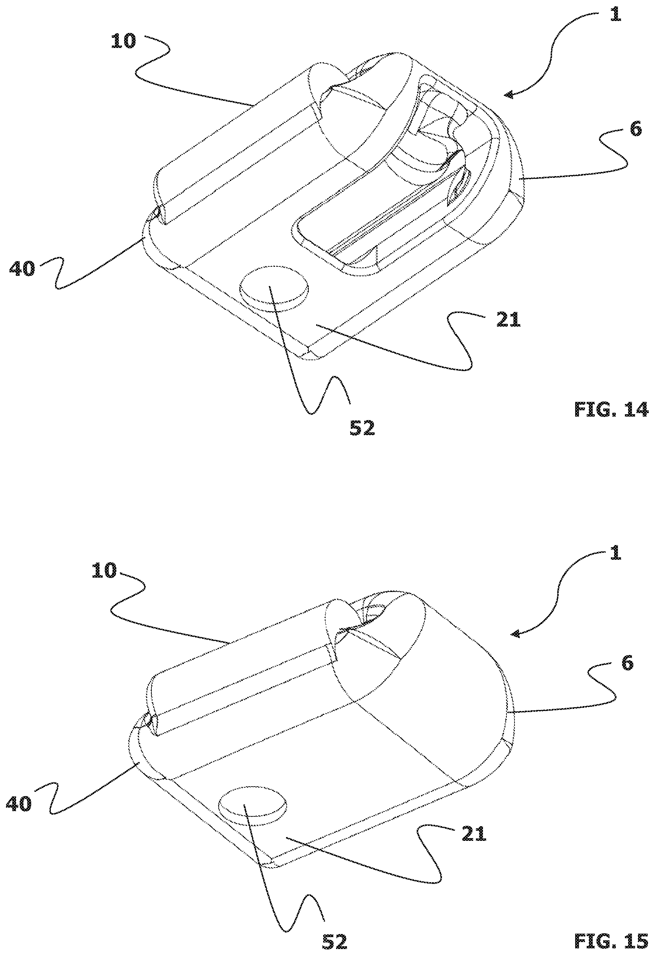

The backsheet (2) has an inlet (5) enabling the connection of a tube (300) for inflating all four inflatable cushions (10, 20, 30, 40) with a pressurizing fluid. Said inlet (5) is preferably located on the outer surface of the backsheet (2) such that the pressurizing fluid enters the urethral cuff (1) through the first portion (22). Accordingly, the pressurizing fluid coming from the tube (300) inflates the first and second inflatable cushions (10, 20) and preferably also the fourth inflatable cushion (40). Said pressurizing fluid is transferred to the third inflatable cushion (30) simultaneously via the conduits (61) in the folding section (6). Said inlet (5) is configured around an inlet hole (52) on the backsheet (2), preferably on the first portion (21) of the backsheet (2) as depicted in Fig. 14 and Fig. 15.

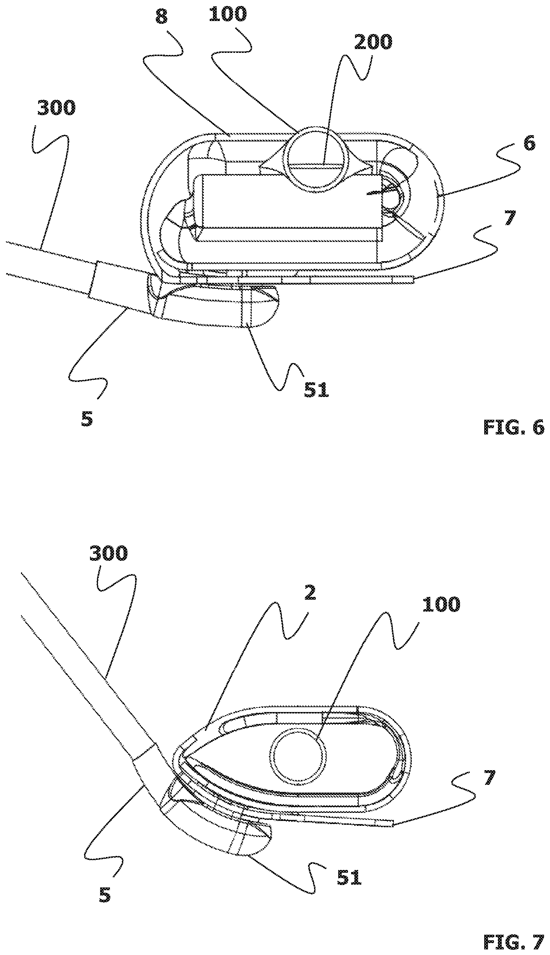

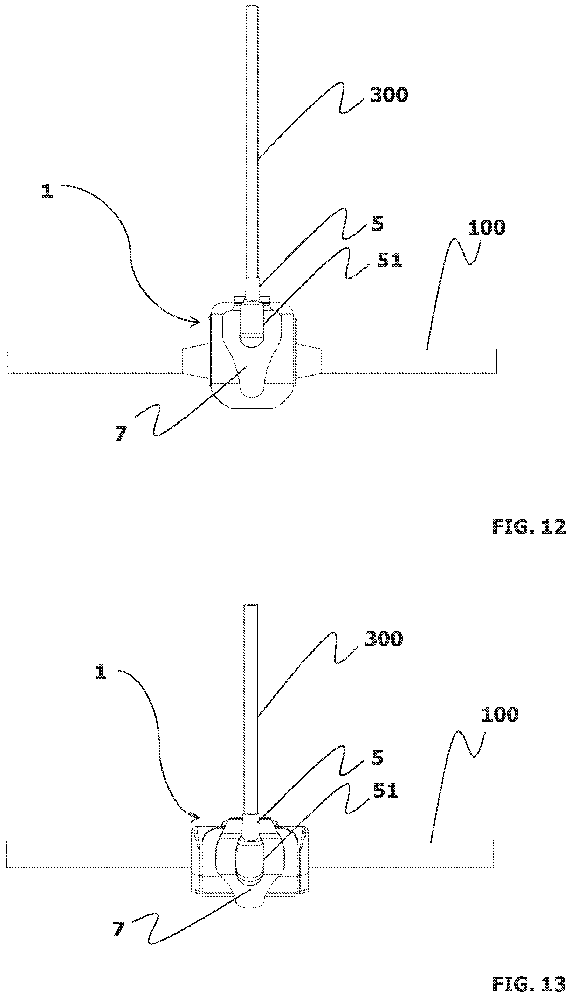

In an embodiment, the backsheet (2) is also provided with a pull tab (7). Said pull tab (7) comprises a connection hole (71) corresponding the inlet (5) when the second portion (22) is folded on top of the first portion (21). Accordingly, the inlet (5) is provided with a protrusion (51) for supporting the pull tab (7) through said hole (71). This configuration ensures that the pull tab (7) is locked around the inlet (5), when the urethral cuff (1) is implanted around the urethra (100) of a patient. Thus, the urethral cuff (1) is more securely fixed on the urethra (100). The top view of this locking mechanism can be seen in Fig. 12 and Fig. 13. Said protrusion (51) is in the form of a cap covering the inlet (5) and forming a cavity to ensure the attachment of the pull tab (7). Fig. 6 and Fig. 7 show the side views of the urethral cuff (1) in its inflated and deflated states, including said protrusion (51) structure.

According to an embodiment of the present invention, the backsheet (2) further has a reinforcement element (8) in the form of a mesh structure. Said reinforcement element (8) covers the backsheet (2) on its outer surface for enhancing strength and lifetime of the urethral cuff (1).

The present invention also proposes an artificial urinary sphincter comprising the urethral cuff (1) according to anyone of the embodiments described above.

According to an embodiment, said artificial urinary sphincter is provided with an electronic system which can be remotely controlled.

When used in an artificial urinary sphincter, said urethral cuff (1) is implanted around the urethra (100) of a patient and operated in coordination with a pump to which it is connected via the tube (300). In this case, said pump is operated by the patient or a physician for enabling the transmission of a pressurizing fluid from a fluid reservoir to said urethral cuff (1). The urethral cuff (1) is inflated as the pressurizing fluid moves into the urethral cuff (1) so as to occlude the urethra (100).

According to a use of the urethral cuff (1) as described by the present invention, when the patient needs to urinate, the patient shall operate the pump to transfer the fluid contained in the urethral cuff (1) into a fluid reservoir which is implanted in the abdomen of the patient and which has a stretchable wall. The pump preferably also prevents the fluid flow towards to the urethral cuff (1) by means of a check valve. As there is no pressurizing fluid in the urethral cuff (1), the patient is now free to urinate.

In the case of the inflation of the urethral cuff (1), the urethral cuff (1) is automatically inflated by the abdominal pressure of the patient as well as by the pressure increase arising from the stretching of the walls of the fluid reservoir. Since the fluid previously found in the urethral cuff (1) was transferred to the stretchable fluid reservoir, the internal pressure of the stretchable fluid reservoir is very high not only because the reservoir is in its stretched form but also because of the abdominal pressure of the patient. Inflation of the urethral cuff (1) occurs gradually because of the fact that the check valve allows a controlled reverse fluid flow towards the urethra (100). Once the controlled flow from the fluid reservoir inflates the urethral cuff (1), the artificial urinary sphincter reaches is stable form. The urethra (100) is now occluded and the patient is continent.

The urethral cuff (1) as described by the present invention overcomes incontinence without damaging the capillaries in the urethra and the corresponding tissue, thus reducing the possibility of atrophy or erosion. Another advantage of the present invention over the prior art is that the urethral cuff (1) according to anyone of the embodiments described above, when deflated, allows insertion of an endoscope or a resectoscope.

## Claims

1. A urethral cuff (1) for use in an artificial urinary sphincter, said urethral cuff having a backsheet (2) forming the body of the urethral cuff (1), said backsheet (2) is composed of a first portion (21) and a second portion (22), the first portion (21) has a first inflatable cushion (10) and a second inflatable cushion (20) which are separated from each other by a gap (3), the second portion (22) has a third inflatable cushion (30); the urethral cuff (1) is **characterized in that**
- said third inflatable cushion (30) is located so as to correspond on top of said gap (3) when the second portion (22) is folded on top of the first portion (21) such that when the urethral cuff (1) is implanted around the urethra (100) of a patient and all three inflatable cushions (10, 20, 30) are inflated, a length (200) of the urethra (100) is flattened among the inflatable cushions (10, 20, 30) resulting in occlusion of the urethra (100),
- said first portion (21) further comprises a fourth inflatable cushion (40) connecting the first and second inflatable cushions (10, 20) such that the gap (3) converts into a recess (4) when all four inflatable cushions (10, 20, 30, 40) are inflated, the recess being surrounded by the first, second and fourth inflatable cushions,
- the third inflatable cushion (30) is sized and shaped to fit into and fill the recess (4) when the second portion (22) is folded on top of the first portion (21) and when all four inflatable cushions (10, 20, 30, 40) are inflated.

2. The urethral cuff (1) according to Claim 1, wherein the flattened length (200) of the urethra (100) forms a U-shaped longitudinal section.

3. The urethral cuff (1) according to Claim 1, wherein the flattened length (200) of the urethra (100) forms a sinusoidal wave shaped longitudinal section.

4. The urethral cuff (1) according to any one of the preceding claims, wherein the urethral cuff (21) further comprises a folding section (6) that allows fluid communication between the first portion (21) and the second portion (22) by means of at least one conduit (61) such that all three first, second, and third inflatable cushions (10, 20, 30) are inflated simultaneously.

5. The urethral cuff (1) according to any one of the preceding claims, wherein the backsheet (2) has an inlet (5) enabling transfer of a pressurizing fluid.

6. The urethral cuff (1) according to Claim 5, wherein said inlet (5) is located on the outer surface of the backsheet (2).

7. The urethral cuff (1) according to Claim 5 or 6, wherein the backsheet (2) is provided with a pull tab (7) having a connection hole (71) located such that the inlet (5) corresponds into said connection hole (71) when the second portion (22) is folded on top of the first portion (21)

8. The urethral cuff (1) according to Claim 7, wherein the inlet (5) is provided with a protrusion (51) for supporting the pull tab (7) through said connection hole (71) such that the pull tab (7) is locked around the inlet (5) upon implantation of the urethral cuff (1) around the urethra (100) of a patient.

9. The urethral cuff (1) according to any one of the preceding claims, wherein the backsheet (2) further has a reinforcement element (8) in the form of a mesh structure.

10. An artificial urinary sphincter comprising the urethral cuff (1) according to Claim 1.

## Patentansprüche

1. Eine Harnröhrenmanschette (1) zur Verwendung in einem künstlichen Harnröhrenschließmuskel, wobei die Harnröhrenmanschette eine Rückwand (2), die den Körper der Harnröhrenmanschette (1) bildet, wobei die Rückwand (2) aus einem ersten Abschnitt (21) und einem zweiten Abschnitt (22) besteht, wobei der erste Abschnitt (21) ein erstes aufblasbares Kissen (10) und ein zweites aufblasbares Kissen (20), die durch einen Spalt (3) voneinander getrennt sind, wobei der zweite Abschnitt (22) ein drittes aufblasbares Kissen (30); die Harnröhrenmanschette (1) ist **dadurch gekennzeichnet, dass**
- das dritte aufblasbare Kissen (30) so angeordnet ist, dass es oben auf dem Spalt (3) entspricht, wenn der zweite Abschnitt (22) über den ersten Abschnitt (21) gefaltet ist, so dass, wenn die Harnröhrenmanschette (1) um die Harnröhre (100) eines und alle drei aufblasbaren Kissen (10, 20, 30) aufgeblasen sind, ein Längenabschnitt (200) der Harnröhre (100) zwischen den aufblasbaren Kissen (10, 20, 30) abgeflacht wird, was zu einer Okklusion der Harnröhre (100) führt,
- wobei der erste Abschnitt (21) ferner ein viertes aufblasbares Kissen (40) umfasst, dass das erste und das zweite aufblasbare Kissen (10, 20) so verbindet, dass der Spalt (3) in eine Aussparung (4) umgewandelt wird, wenn alle vier aufblasbaren Kissen (10, 20, 30, 40) aufgeblasen sind, wobei die Aussparung von dem ersten, zweiten und vierten aufblasbaren Kissen umgeben ist,
- wobei das dritte aufblasbare Kissen (30) so dimensioniert und geformt ist, dass es in die Aussparung (4) passt und diese ausfüllt Aussparung (4) passt und diese ausfüllt, wenn der zweite Abschnitt (22) über den ersten Abschnitt (21) gefaltet ist und alle vier aufblasbaren Kissen (10, 20, 30, 40) aufgeblasen sind.

2. Die Harnröhrenmanschette (1) gemäß Anspruch 1, wobei die abgeflachte Länge (200) der Harnröhre (100) einen U-förmigen Längsabschnitt bildet.

3. Die Harnröhrenmanschette (1) gemäß Anspruch 1, wobei die abgeflachte Länge (200) der Harnröhre (100) einen sinusförmigen Längsabschnitt bildet.

4. Die Harnröhrenmanschette (1) gemäß einem der vorstehenden Ansprüche, wobei die Harnröhrenmanschette (21) ferner einen Faltabschnitt (6) umfasst, der eine Flüssigkeitskommunikation Verbindung zwischen dem ersten Abschnitt (21) und dem zweiten Abschnitt (22) mittels mindestens einer Leitung (61) ermöglicht, so dass alle drei ersten, zweiten und dritten aufblasbaren Kissen (10, 20, 30) gleichzeitig aufgeblasen werden.

5. Die Harnröhrenmanschette (1) gemäß einem der vorstehenden Ansprüche, wobei die Rückwand (2) einen Einlass (5) aufweist, der die Übertragung eines Druckfluids ermöglicht.

6. Die Harnröhrenmanschette (1) gemäß Anspruch 5, wobei sich der Einlass (5) auf der Außenseite der Rückwand (2) angeordnet ist.

7. Die Harnröhrenmanschette (1) gemäß Anspruch 5 oder 6, wobei die Rückseite (2) mit einer Zuglasche (7) versehen ist, die ein Verbindungsloch (71) aufweist, das so angeordnet ist, dass der Einlass (5) mit dem Verbindungsloch (71) übereinstimmt, wenn der zweite Abschnitt (22) auf den ersten Abschnitt (21) gefaltet wird

8. Die Harnröhrenmanschette (1) gemäß Anspruch 7, wobei der Einlass (5) mit einem Vorsprung (51) zum Halten der Zuglasche (7) durch das Verbindungsloch (71) vorgesehen ist, so dass die Zuglasche (7) bei der Implantation der Harnröhrenmanschette (1) um den Einlass (5) herum arretiert wird Harnröhrenmanschette (1) um die Harnröhre (100) eines Patienten herum verriegelt wird.

9. Die Harnröhrenmanschette (1) gemäß einem der vorstehenden Ansprüche, wobei die Rückwand (2) ferner ein Verstärkungselement (8) in Form einer Netzstruktur.

10. Künstlicher Harnröhrenschließmuskel, umfassend die Harnröhrenmanschette (1) gemäß Anspruch 1.

## Revendications

1. - Manchon urétral (1) destiné à être utilisé dans un sphincter urinaire artificiel, ledit manchon urétral ayant une feuille de support (2) formant le corps du manchon urétral (1), ladite feuille de support (2) étant composée d'une première partie (21) et d'une seconde partie (22), la première partie (21) ayant un premier coussin gonflable (10) et un deuxième coussin gonflable (20) qui sont séparés l'un de l'autre par un espacement (3), la seconde partie (22) ayant un troisième coussin gonflable (30) ; le manchon urétral (1) étant **caractérisé par le fait que**
- ledit troisième coussin gonflable (30) est situé de façon à correspondre au dessus dudit espacement (3) lorsque la seconde partie (22) est repliée au-dessus de la première partie (21) de telle sorte que, lorsque le manchon urétral (1) est implanté autour de l'urètre (100) d'un patient et que l'ensemble des trois coussins gonflables (10, 20, 30) sont gonflés, une longueur (200) de l'urètre (100) est aplatie entre les coussins gonflables (10, 20, 30), ce qui conduit à une occlusion de l'urètre (100),
- ladite première partie (21) comprend en outre un quatrième coussin gonflable (40) reliant les premier et deuxième coussins gonflables (10, 20) de telle sorte que l'espacement (3) se convertit en un renfoncement (4) lorsque l'ensemble des quatre coussins gonflables (10, 20, 30, 40) sont gonflés, le renfoncement étant entouré par les premier, deuxième et quatrième coussins gonflables,
- le troisième coussin gonflable (30) est dimensionné et façonné pour s'encastrer dans le renfoncement (4) et le remplir lorsque la seconde partie (22) est repliée au-dessus de la première partie (21) et lorsque l'ensemble des quatre coussins gonflables (10, 20, 30, 40) sont gonflés.

2. - Manchon urétral (1) selon la revendication 1, dans lequel la longueur aplatie (200) de l'urètre (100) forme une section longitudinale en forme de U.

3. - Manchon urétral (1) selon la revendication 1, dans lequel la longueur aplatie (200) de l'urètre (100) forme une section longitudinale en forme d'onde sinusoïdale.

4. - Manchon urétral (1) selon l'une quelconque des revendications précédentes, dans lequel le manchon urétral (21) comprend en outre une section de pliage (6) qui permet une communication fluidique entre la première partie (21) et la seconde partie (22) au moyen d'au moins un conduit (61) de telle sorte que l'ensemble des trois premier, deuxième et troisième coussins gonflables (10, 20, 30) sont gonflés simultanément.

5. - Manchon urétral (1) selon l'une quelconque des revendications précédentes, dans lequel la feuille de support (2) a une entrée (5) permettant un transfert d'un fluide de pressurisation.

6. - Manchon urétral (1) selon la revendication 5, dans lequel ladite entrée (5) est située sur la surface externe de la feuille de support (2).

7. - Manchon urétral (1) selon la revendication 5 ou 6, dans lequel la feuille de support (2) comporte une patte de traction (7) ayant un trou de liaison (71) situé de telle sorte que l'entrée (5) corresponde audit trou de liaison (71) lorsque la seconde partie (22) est repliée au-dessus de la première partie (21).

8. - Manchon urétral (1) selon la revendication 7, dans lequel l'entrée (5) comporte une protubérance (51) pour porter la patte de traction (7) à travers ledit trou de liaison (71) de telle sorte que la patte de traction (7) soit verrouillée autour de l'entrée (5) lors de l'implantation du manchon urétral (1) autour de l'urètre (100) d'un patient.

9. - Manchon urétral (1) selon l'une quelconque des revendications précédentes, dans lequel la feuille de support (2) a en outre un élément de renfort (8) sous la forme d'une structure maillée.

10. - Sphincter urinaire artificiel comprenant le manchon urétral (1) selon la revendication 1.
